# EUROPEAN PATENT SPECIFICATION

(11) **EP 3 915 630 B1**
(45) Date of publication and mention of the grant of the patent: **30.10.2024**
(21) Application number: 19911977.7
(22) Date of filing: 12.02.2019
(51) Int. Cl.: A61N 2/00, A61N 2/02, A61N 1/04, A61N 1/36

(54) **BRAIN STIMULATION DEVICE**
VORRICHTUNG ZUR STIMULATION DES GEHIRNS
DISPOSITIF DE STIMULATION CÉRÉBRALE

(30) Priority: 24.01.2019 KR 20190009225
(43) Date of publication of application: 01.12.2021
(73) Proprietor: BBB Inc., Seongnam-si, Gyeonggi-do 13522 (KR)
(72) Inventor: CHOI, Jae Kyu, Seoul 06194 (KR); CHOI, Eun Jong, Daejeon 35246 (KR); YUN, Kyong Sik, Seoul 03362 (KR); HWANG, Hyun Doo, Seongnam-si Gyeonggi-do 13491 (KR)
(74) Representative: M. Zardi & Co S.A.
(86) International application number: PCT/KR2019/001704
(87) International publication number: WO 2020/153524

(56) References cited:
- CN-A- 105 342 603
- KR-A- 20140 080 299
- KR-A- 20160 086 275
- KR-A- 20160 095 579
- KR-B1- 101 290 724
- KR-Y1- 200 429 471
- US-A1- 2012 083 717
- US-A1- 2017 215 753
- US-A1- 2018 256 887
- US-A1- 2018 310 855

## Description

### [Technical Field]

The present invention relates to a brain stimulation device, and more particularly, to a brain stimulation device which a user can conveniently wear without a complicated manipulation for closely attaching an electrode to his/her head.

### [Background Art]

A brain as an internal organ of a human head is a highest central organ of a nervous system, and is divided into cerebrums, cerebellum, midbrain, pons, and medulla. Further, the brain generates a brainwave which is a signal indicating the sum of neural activity levels measured in the epidermis of the brain.

Examples of a method for measuring a state of the brain include an electroencephalogram inspection (EEG) that inspects the state of the brain by measuring the brainwave received from an electrode by mounting a pad having the electrode on the epidermis, a CT inspection that inspects the state of the brain by performing tomography of the brain at various angles by using radiation or ultrasonic waves, an MRI inspection that photographs the brain by magnetic resonance, and the like.

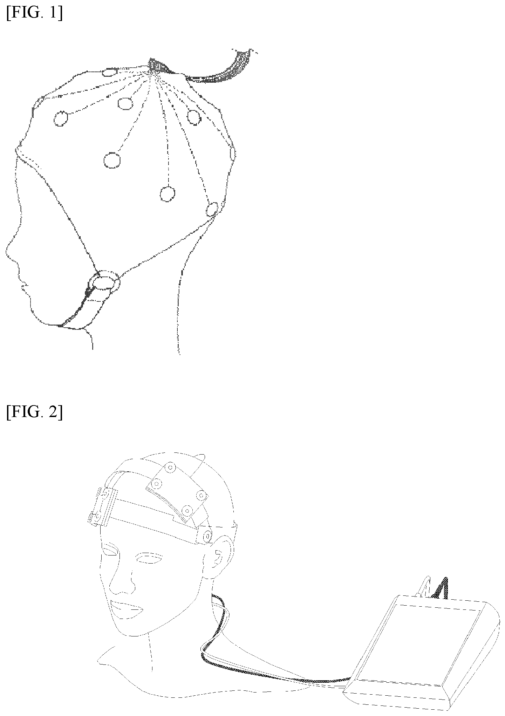

FIG. 1 illustrates an example of a conventional EEG inspection.

In the EEG inspection, a swimming cap type elastic measuring device is worn and the electrodes are attached one by one, which requires a long measurement preparation time.

Meanwhile, various concepts are known in the field of neural stimulation of brain structures, and brain stimulation which stimulates the brain to achieve a predetermined object is largely divided into invasive brain stimulation and non-invasive brain stimulation.

The invasive brain stimulation is a method that penetrates an electrode into the brain through surgery and applies an electrical signal thereto, and the non-invasive brain stimulation is a method that achieves a predetermined effect by stimulating the brain without invading an electrode into cranium.

Examples of specific brain stimulation include deep electrical stimulation, transcranial magnetic stimulation (TMS), and transcranial electrical stimulation (TES), and examples of the transcranial electrical stimulation (TES) include transcranial direct current stimulation (tDCS), transcranial alternating current stimulation (tACS), and transcranial random noise stimulation (tRNS).

FIG. 2 illustrates an example of conventional transcranial direct current stimulation (tDCS).

In the case of the transcranial direct current stimulation (tDCS), a weak continuous direct current is applied through two large-sized electrodes provided on the epidermis. This causes a small change in membrane potentials of nerve cells of a cortex and a change in firing rate, and as a result, an excitement level of the nerve cell is influenced.

Referring to FIG. 2, an electrode is fixed by using a headband. Since people have different head shapes and sizes, a band which is adjustable in length or stretchable should be used.

However, such a swimming cap or headband type brain stimulation device is vulnerable to contamination and weak in durability, and requires a long measurement preparation time. US 2018256887 A1 discloses an electrical stimulation system including one or more of an electrode assembly including one or more electrodes and an electronics subsystem. US 2017215753 A1 discloses a wearable device, which is worn on a user's head to apply an electrical stimulation to a brain or measure brain waves from the brain, comprising: a wearing identification unit for identifying a worn state of the wearable device using a first sensor module detecting the worn state of the wearable device of a user; an electrode unit for applying an electrical stimulation to a user's brain or measuring brain waves from the user's brain; and a control unit for controlling the electrode unit to start the electrical stimulation or a brain wave measurement on the basis of the identification result by the wearing identification unit. US 2018310855 A1 discloses a wearable brain activity device with electrodes held on a person's head by a frame comprising a ring portion which encircles the person's head and an arc portion which loops over the top of the person's head.

### [Disclosure]

### [Technical Problem]

Therefore, an object to be achieved by the present invention is to provide a brain stimulation device which a user can conveniently wear without a complicated manipulation for closely attaching an electrode to his/her head.

### [Technical Solution]

In order to achieve the object, the present invention provides a brain stimulation device according to claim 1. Embodiments of the invention are defined in the dependent claims.

Meanwhile, when the two electromagnets constituting the electrodes are the same stimulus, the electromagnets may stimulate a deep place of the head of the user, and when the two electromagnets constituting the electrodes are different stimuli, the electromagnets may stimulate a shallow place of the head of the user.

According to another embodiment of the present invention, each of the electrodes of the electrode unit may be located in a cap expanded by air pressure, and the cap may be expanded to closely attach the electrode unit to the head.

Further, a pressure sensor may be provided on one end of the electrode unit contacting the head, and the electrode unit may move to the head until a pressure of the pressure sensor becomes a predetermined pressure or higher.

According to still another embodiment of the present invention, the brain stimulation device may further include an electrode connected to the donut type support unit by a band and disposed inside the donut type support unit, in which when the donut type support unit surrounds the head, the electrodes may be closely attached onto the head.

### [Advantageous Effects]

According to the present invention, a user can conveniently wear a brain stimulation device a complicated manipulation for closely attaching an electrode to his/her head.

Further, according to the present invention, the electrode is closely attached to the head, and then pressed through pneumatic expansion, such that the brain stimulation device can be worn regardless of the size of a user's head.

### [Description of Drawings]

FIG. 1 illustrates an example of a conventional EEG inspection.
FIG. 2 illustrates an example of conventional transcranial direct current stimulation (tDCS).
FIG. 3 is a configuration diagram of a brain stimulation device according to an embodiment of the present invention.
FIG. 4 illustrates a polarity change pattern when polarities of electrodes constituting an electrode unit 310 are constituted by two polarities.
FIG. 5 illustrates a direction of a magnetic flux of the electrode unit 310 for each mode.
FIG. 6 is a configuration diagram of a brain stimulation device according to another embodiment of the present invention.
FIG. 7 is a configuration diagram of a brain stimulation device according to still another embodiment of the present invention.
FIG. 8 illustrates a state in which a brain stimulation device according to an embodiment of the present invention is worn on a head of a user.

### [Best Mode]

In order to achieve the object, the present invention provides a brain stimulation device including: an electrode unit constituted by one or more electrodes and configured to move back and forth to a head of a user to fix the electrode onto the head of the user; an auxiliary pressing unit configured to move back and forth to the head of the user to fix the electrode unit onto the head of the user; and a donut type support unit having a donut type inner side on which the electrode unit and the auxiliary pressing unit are disposed and which surrounds the head of the user.

### [Mode for Invention]

Hereinafter, embodiments of the present invention will be described in detail with reference to the accompanying drawings so that those skilled in the art can easily carry out the present invention. However, the embodiments are provided for more specifically describing the present invention, and it will be obvious to those skilled in the art that the scope of the present invention is not limited thereto.

The configuration of the present invention for clarifying a solution to the problem to be solved by the present invention will be described in detail with reference to the accompanying drawings based on embodiments of the present invention. It is to be noted that in assigning reference numerals to the components of the drawings, the same reference numerals are assigned to the same components even though the same components are illustrated on different drawings, and components of other drawings may be cited when a certain drawing needs to be explained. In addition, when it is determined that detailed descriptions of publicly-known functions or configurations related to the present invention and the other general matters may unnecessarily obscure the subject matter of the present invention in describing the operating principle for the embodiment of the present invention in detail, the detailed descriptions will be omitted.
Further, throughout the specification, when it is described that a part is "connected" with another part, it not only means that the parts are "directly connected" to each other, and but also means that the parts are "indirectly connected" to each other with another element interposed therebetween. In this specification, the singular form also includes the plural form, unless the context indicates otherwise. The term "comprise" or "comprising" used in the specification indicates the inclusion of a stated component, step, operation or element, but does not exclude the presence or addition of one or more other components, steps, operations, or elements.

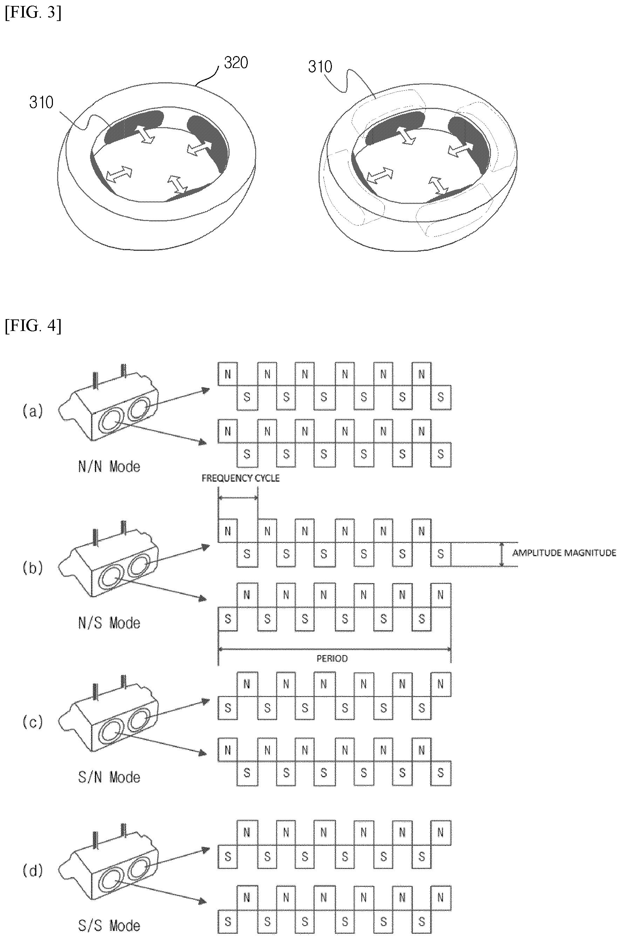

FIG. 3 is a configuration diagram of a brain stimulation device according to an embodiment of the present invention.

Referring to FIG. 3, the brain stimulation device according to the present embodiment is constituted by an electrode unit 310 and a donut type support unit 320.
The electrode unit 310 is constituted by four or more electrodes, and in order to closely attach the electrodes to a head of a user, the electrodes move back and forth from an inner side of the donut type support unit 320 to the head.

Referring to FIG. 3(a), four electrodes are disposed on the inner side of the donut type support unit 320 and while moving back and forth, the electrodes are closely attached to the head.

FIG. 3(b) illustrates a state in which fourth electrodes are inserted into the inside of the donut type support unit 320.

Respective electrodes of the electrode unit 310 are located in a cap expanded by air pressure, and the electrode unit 310 may be closely attached to the head by moving the electrodes back and forth and expanding the cap.

The donut type support unit 320 has a donut type inner side on which the electrode unit 310 and an auxiliary pressing unit 320 are disposed and which surrounds the head.

Further, as another embodiment, a pressure sensor may be provided on one end of the electrode unit 310 contacting the head, and the electrode unit 310 may move to the head until a pressure of the pressure sensor becomes a predetermined pressure or higher.

The respective electrodes constituting the electrode unit 310 may be closely attached to the head to apply the pressure to the head, by moving the electrodes and closely attaching the electrodes to the head until a pressure measured by the pressure sensor provided in each of the electrodes becomes a predetermined threshold or higher, or by increasing the air pressure in the cap including the electrodes.

Air may be supplied to or discharged from the electrode unit 310 by using an air pump, such that back and forth movement of the electrode unit 310 or expansion or contraction of the cap is made. In order to closely attach the electrode unit 310 to the head, the air may be supplied to the electrode unit 310 through the air pump, and simultaneously, an air valve may be closed, and an operation of the air pump may be stopped and the air valve may be opened in order to return the electrode unit 310 to its original position. The cap including the electrode may also be expanded and contracted by using the air pump and the air valve.

As another embodiment, a user may manually determine the back and forth movement of the electrodes of the electrode unit 310 and the increase or decrease of the air pressure.

FIG. 4 illustrates a polarity change pattern when polarities of electrodes constituting the electrode unit 310 are constituted by two polarities.

The electrodes constituting the electrode unit 310 may be configured by electromagnets having magnetic properties of an N pole and an S pole by a flow of current. The electrode unit 310 is constituted by one or more electromagnets, but according to the invention two electromagnets are provided.

The electrodes illustrated in FIG. 4 are constituted by two electromagnets, and each electromagnet is changed to the N pole and the S pole over time, thereby applying a stimulus to the head of the user.

When two electromagnets constitute the electrode unit 310, the magnetic property of each electromagnet may be changed to an N/N mode, an N/S mode, an S/N mode, and an S/S mode.

FIG. 4(a) illustrates the N/N mode, FIG. 4(b) illustrates the N/S mode, FIG. 4(c) illustrates the S/N mode, and FIG. 4(d) illustrates the S/S mode.

Various modified embodiments of each mode are available according to an amplitude magnitude, a stimulation period, and a frequency cycle. Table 1 shows the stimulation period, a frequency, an amplification magnitude, and an available mode in each electrode.

**[Table 1]**

| | Front electrode | Rear electrode | Left electrode | Right electrode |
|---|---|---|---|---|
| Stimulation period | 1.0 to 120 min | | | |
| Frequency | 0.1 to 3,000 Hz | | | |
| Amplification magnitude | 0.1 to 100 mT | 0.1 to 100 mT | 0.1 to 100 mT | 0.1 to 100 mT |
| Mode | N/N, N/S, S/N, S/S | N/N, N/S, S/N, S/S | N/N, N/S, S/N, S/S | N/N, N/S, S/N, S/S |

In the case of the N/N mode or the S/S mode, the magnitude of the magnetic flux output from the electrode unit 310 may be enhanced, such that the magnetic flux may stimulate a deep place of the head, and in the case of the N/S mode or the S/N mode, the magnetic flux output from the N pole of the electrode unit 310 may be immediately input into the S pole located next to the N pole, such that the magnetic flux may stimulate a shallow place of the head. That is, when two electromagnets constituting the electrodes are the same stimulus, the electromagnets may stimulate the deep place of the head of the user, and when two electromagnets constituting the electrodes are different stimuli, the electromagnets may stimulate the shallow place of the head of the user.

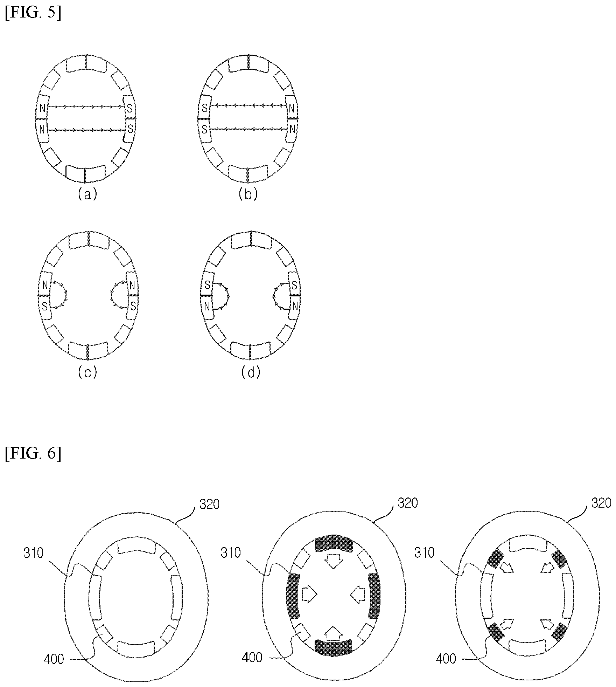

FIG. 5 illustrates a direction of a magnetic flux of the electrode unit 310 for each mode.

FIGS. 5(a) and 5(b) illustrate a magnetic flux connected from the electrode in the N/N mode to the electrode in the S/S mode.

FIGS. 5(c) and 5(d) illustrate that a magnetic flux output from the N pole is connected to the S pole located next to the N pole in the N/S mode or the S/N mode.

Referring to FIG. 5, it can be seen that in the N/N mode or the S/S mode, the magnetic flux reaches the deep place of the head to stimulate the deep place of the head, and it can be seen that in the N/S mode or the S/N mode, a sufficient magnetic flux may not reach the deep place of the head, and the magnetic flux is formed in a lower portion of the head.

FIG. 6 is a configuration diagram of a brain stimulation device according to another embodiment of the present invention.

Referring to FIG. 6, the brain stimulation device according to the present embodiment is constituted by the electrode unit 310, the donut type support unit 320, and the auxiliary pressing unit 400.

The descriptions of the electrode unit 310 and the donut type support unit 320 are duplicated with those in FIG. 3 and thus will be omitted.

The auxiliary pressing unit 400 is located between the electrodes of the electrode unit 310, and moves back and forth to the head from the inner side of the donut type support unit 320 in order to closely attach the electrode unit 310 to the head of the user.

Respective individual pressing units of the auxiliary pressing unit 400 are located in the cap expanded by the air pressure, and the auxiliary pressing unit 400 may be more closely attached to the head by moving the individual pressing units back and forth, and expanding the cap, as compared to when only the electrode unit 310 is used. The brain stimulation device according to the present invention may be more stably fixed to the head through the auxiliary pressing unit 400.

Further, as another embodiment, a pressure sensor may be provided on one end of the auxiliary pressing unit 400 contacting the head, and the auxiliary pressing unit 400 may move to the head until a pressure of the pressure sensor becomes a predetermined pressure or higher.

Until the pressure measured by the pressure sensor provided in each of the individual pressing units becomes a predetermined threshold or more, the respective individual pressing units constituting the auxiliary pressing unit 400 may be closely attached to the head to apply the pressure to the head. In this case, it is possible to further closely attach the individual pressing units or increase the air pressure in order to increase the pressure.

Meanwhile, the user may manually determine the back and forth movement of the individual pressing units of the auxiliary pressing unit 400, and the increase of the air pressure.

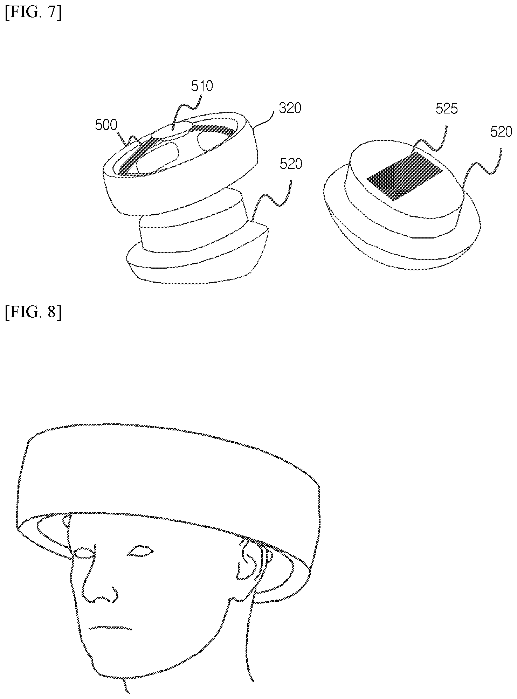

FIG. 7 is a configuration diagram of a brain stimulation device according to still another embodiment of the present invention.

The brain stimulation device illustrated in FIG. 7 is configured to further include a connection unit 500, an upper electrode 510, and a cradle 520.

The connection unit 500 connects the upper electrode 510 and the donut type support unit 320. The connection unit 500 is formed by a stretchable band, and when the head of the user and the upper electrode 510 are in contact with each other, the connection unit 500 changes a position of the upper electrode 510 in a predetermined range and enables the brain stimulation device to be stably worn on the head.

When the user wears the brain stimulation device, the upper electrode 510 is an electrode that applies a stimulus to an upper portion of the head. The upper electrode 510 is connected to the donut type support unit 320 by the connection unit 500.

The cradle 520 is a device that keeps the brain stimulation device when the user does not use the brain stimulation device. Since the donut type support unit 320 is a donut type, a protrusion 522 is formed in the cradle 520 so that the brain stimulation device is suspended through an empty space at the center of the donut type support unit 320.

The cradle 520 includes a display unit 525, and the display unit 525 displays back and forth movement distances of the electrode unit 310 and the auxiliary pressing unit 400, the air pressure, etc. Further, the display unit 525 is configured by a touch screen, and the brain stimulation device may be controlled by touching the display unit 525.

FIG. 8 illustrates a state in which a brain stimulation device according to an embodiment of the present invention is worn on a head.

Referring to FIG. 8, it can be seen that the brain stimulation device can be conveniently worn like the swimming cap type illustrated in FIG. 1, and can closely attach the electrode to the head unlike the headband type illustrated in FIG. 2.
By using the brain stimulation device according to the present invention, transcranial magnetic stimulation (TMS) and repetitive transcranial magnetic stimulation (rIMS) may be performed.

When the brain is stimulated, the brain exhibits an excitability or inhibiting effect through a series of complicated processes, and a technical principle of the transcranial magnetic stimulation (TMS) is to cause a magnetic field which is abruptly changed by making a short and strong current flow on a coil, and such a magnetic field causes an electric field again to excite nerve cells of the cerebrum when stimulating an upper portion of the epidermis of a person.

The repetitive transcranial magnetic stimulation (rTMS) is a method that causes an evoked potential by continuous magnetic stimuli to activate the nerve cells of a cerebral cortex. When the continuous stimuli are applied with various frequencies and strengths, the continuous stimuli may cause the excitability or inhibiting effect for the cerebral cortex, and change a cerebral cortex activity in a short or long term. A lowfrequency stimulus of one time per second, i.e., 1 Hz stimulus inhibits the activity of a motor cortex and a high-frequency stimulus of 10 times or 20 times per second, i.e., 10 or 20 Hz stimulus increases the cerebral cortex activity in a short term, which makes it possible to provide various effects. By using such effects, the brain stimulation device may be attempted to a variety of clinical diseases including stroke, depression, Parkinson's disease, epilepsy, pain, and the like.

As described above, the present invention has been described with reference to specified matters such as detailed components, and limited embodiments and drawings, but the embodiments are just provided to assist more overall understanding of the present invention, the present invention is not limited to the embodiments, and various modifications and changes can be made which fall under the scope of the claims.

## Claims

1. A brain stimulation device comprising:
an electrode unit (310) constituted by two electromagnets and configured to move back and forth to a head of a user to fix the electrodes onto the head of the user;
an auxiliary pressing unit (400) configured to move back and forth to the head of the user to fix the electrode unit (310) onto the head of the user; and
a support unit (320) having an inner side on which the electrode unit (310) and the auxiliary pressing unit (400) are disposed and which surrounds the head of the user,
**characterized in that** the magnetic property of the electrode unit (310) is adapted to be changed to an N/N mode, an N/S mode, an S/N mode, and an S/S mode,
wherein, in the case of the N/N mode or the S/S mode, the magnitude of the magnetic flux output from the electrode unit(310) is enhanced, such that the magnetic flux is configured to stimulate a deep place of the head, and in the case of the N/S mode or the S/N mode, the magnetic flux output from the N pole of the electrode unit(310) is immediately input into the S pole located next to the N pole, such that the magnetic flux is configured to stimulate a shallow place of the head,
wherein the auxiliary pressing unit (400) is located between the electrodes of the electrode unit (310), and
wherein the stimulation period of each electrode is within a range of 1.0 to 120 minutes, and
wherein the stimulus frequency of each electrode is within a range of 0.1 to 3,000 Hz, and
wherein the magnitude of the magnetic flux of each electrode is within a range of 0.1 to 100 mT.

2. The brain stimulation device of claim 1, wherein each of the electrodes of the electrode unit (310) is located in a cap expanded by air pressure, and
the electrode unit (310) is closely attached to the head by expanding the cap.

3. The brain stimulation device of claim 1, wherein a pressure sensor is provided on one end of the electrode unit (310) contacting the head, and
the electrode unit (310) is configured to move to the head until a pressure of the pressure sensor becomes a predetermined pressure or higher.

4. The brain stimulation device of claim 1, further comprising:
an electrode connected to the support unit (320) by a band and disposed inside the support unit (320),
wherein when the support unit (320) surrounds the head, the electrodes (510) are closely attached onto the head.

## Patentansprüche

1. Hirnstimulationsvorrichtung umfassend:
eine Elektrodeneinheit (310), die durch zwei Elektromagneten gebildet ist und dazu konfiguriert ist, sich zu einem Kopf eines Benutzers vor und zurück zu bewegen, um die Elektroden an dem Kopf des Benutzers zu fixieren;
eine Hilfspresseinheit (400), die dazu konfiguriert ist, sich zu dem Kopf des Benutzers vor und zurück zu bewegen, um die Elektrodeneinheit (310) an dem Kopf des Benutzers zu fixieren; und
eine Trägereinheit (320), die eine Innenseite aufweist, an welcher die Elektrodeneinheit (310) und die Hilfspresseinheit (400) angeordnet sind und welche den Kopf des Benutzers umgibt,
**dadurch gekennzeichnet, dass** die magnetische Eigenschaft der Elektrodeneinheit (310) daran angepasst ist, zu einem N/N-Modus, einem N/S-Modus, einem S/N-Modus und einem S/S-Modus geändert zu werden,
wobei im Falle des N/N-Modus oder des S/S-Modus die Größe des von der Elektrodeneinheit (310) ausgegebenen Magnetflusses verstärkt wird, so dass der Magnetfluss dazu konfiguriert ist, eine tiefe Stelle des Kopfes zu stimulieren, und im Falle des N/S-Modus oder des S/N-Modus der von dem N-Pol der Elektrodeneinheit (310) ausgegebene Magnetfluss sofort in den nahe dem N-Pol befindlichen S-Pol eingegeben wird, so dass der Magnetfluss dazu konfiguriert ist, eine flache Stelle des Kopfes zu stimulieren,
wobei die Hilfspresseinheit (400) sich zwischen den Elektroden der Elektrodeneinheit (310) befindet, und
wobei die Stimulationsperiode jeder Elektrode innerhalb eines Bereichs von 1,0 bis 120 Minuten ist, und
wobei die Stimulationsfrequenz jeder Elektrode innerhalb eines Bereichs von 0,1 bis 3000 Hz ist, und
wobei die Größe des Magnetflusses jeder Elektrode innerhalb eines Bereichs von 0,1 bis 100 mT ist.

2. Hirnstimulationsvorrichtung nach Anspruch 1, wobei jede der Elektroden der Elektrodeneinheit (310) sich in einer durch Luftdruck expandierten Kappe befindet, und
die Elektrodeneinheit (310) durch Expandieren der Kappe eng an dem Kopf angebracht wird.

3. Hirnstimulationsvorrichtung nach Anspruch 1, wobei ein Drucksensor an einem den Kopf kontaktierenden Ende der Elektrodeneinheit (310) vorgesehen ist, und
die Elektrodeneinheit (310) dazu konfiguriert ist, sich zu dem Kopf zu bewegen, bis ein Druck des Drucksensors ein vorgegebener Druck oder höher wird.

4. Hirnstimulationsvorrichtung nach Anspruch 1, ferner umfassend:
eine Elektrode, die mit der Trägereinheit (320) durch ein Band verbunden ist und innerhalb der Trägereinheit (320) angeordnet ist,
wobei, wenn die Trägereinheit (320) den Kopf umgibt, die Elektroden (510) eng an dem Kopf angebracht sind.

## Revendications

1. Dispositif de stimulation cérébrale comprenant :
une unité d'électrodes (310) constituée de deux électroaimants et configurée pour se déplacer d'avant en arrière vers la tête d'un utilisateur afin de fixer les électrodes sur la tête de l'utilisateur ;
une unité de pression auxiliaire (400) configurée pour se déplacer d'avant en arrière vers la tête de l'utilisateur afin de fixer l'unité d'électrodes (310) sur la tête de l'utilisateur ; et
une unité de support (320) présentant un côté interne sur lequel sont disposées l'unité d'électrodes (310) et l'unité de pression auxiliaire (400) et qui entoure la tête de l'utilisateur,
**caractérisé en ce que** la propriété magnétique de l'unité d'électrodes (310) est adaptée pour être modifiée en un mode NIN, un mode N/S, un mode S/N, et un mode S/S,
dans lequel, dans le cas du mode N/N ou du mode S/S, l'intensité du flux magnétique délivré par l'unité d'électrodes (310) est augmentée, de telle sorte que le flux magnétique est configuré pour stimuler un endroit profond de la tête, et dans le cas du mode N/S ou du mode S/N, le flux magnétique délivré par le pôle N de l'unité d'électrodes (310) est immédiatement introduit dans le pôle S situé à côté du pôle N, de telle sorte que le flux magnétique est configuré pour stimuler un endroit peu profond de la tête,
dans lequel l'unité de pression auxiliaire (400) est située entre les électrodes de l'unité d'électrodes (310), et
dans lequel la période de stimulation de chaque électrode est dans une plage de 1,0 à 120 minutes, et dans lequel la fréquence de stimulation de chaque électrode est dans une plage de 0,1 à 3000 Hz, et
dans lequel l'intensité du flux magnétique de chaque électrode est dans une plage de 0,1 à 100 mT.

2. Dispositif de stimulation cérébrale selon la revendication 1, dans lequel chacune des électrodes de l'unité d'électrodes (310) est située dans une coiffe dilatée par une pression d'air, et
l'unité d'électrodes (310) est étroitement attachée à la tête par la dilatation de la coiffe.

3. Dispositif de stimulation cérébrale selon la revendication 1, dans lequel un capteur de pression est fourni sur une extrémité de l'unité d'électrodes (310) en contact avec la tête, et
l'unité d'électrodes (310) est configurée pour se déplacer vers la tête jusqu'à ce qu'une pression du capteur de pression soit supérieure ou égale à une pression prédéterminée.

4. Dispositif de stimulation cérébrale selon la revendication 1, comprenant en outre :
une électrode reliée à l'unité de support (320) par une bande et disposée à l'intérieur de l'unité de support (320),
dans lequel lorsque l'unité de support (320) entoure la tête, les électrodes (510) sont étroitement attachées sur la tête.
